(19) Europäisches Patentamt
European Patent Office
Office européen des brevets

(11) Numéro de publication: **0 125 937**
**A2**

(12) # DEMANDE DE BREVET EUROPEEN

(21) Numéro de dépôt: 84400485.3

(22) Date de dépôt: 09.03.84

(51) Int. Cl.$^3$: C 07 D 233/06
C 07 D 233/22, A 61 K 31/415

(30) Priorité: 18.03.83 FR 8304524

(43) Date de publication de la demande: 21.11.84 Bulletin 84/47

(84) Etats contractants désignés: AT BE CH DE FR GB IT LI LU NL SE

(71) Demandeur: SYNTHELABO
58, rue de la Glacière
F-75621 Paris Cedex 13(FR)

(72) Inventeur: Bigg, Dennis
5, Parc de Diane
F-78350 Jouy en Josas(FR)

(72) Inventeur: Menin, Jacques
Cité Balzac 118, rue Balzac
D.114-94400 Vitry Sur Seine(FR)

(74) Mandataire: Ludwig, Jacques et al,
SYNTHELABO Service Brevets 58, rue de la Glacière
F-75621 Paris Cedex 13(FR)

(54) **Dérivés d'(indényl-2)-2 imidazoline, procédé pour les préparer, et compositions pharmaceutiques qui les contiennent.**

(57) Composés répondant à la formule générale I

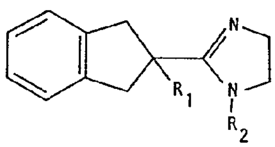

(I)

dans laquelle

. $R_1$ est un atome d'hydrogène ou un groupe $(C_1-C_4)$alkyle, benzyle, phénéthyle ou méthoxy, et

$R_2$ est un atome d'hydrogène ou un groupe méthyle,

ainsi que leurs sels d'addition à des ascides pharmaceutiquement acceptables.

Application en thérapeutique.

EP 0 125 937 A2

Croydon Printing Company Ltd.

DERIVES D'(INDENYL-2)-2 IMIDAZOLINE, PROCEDE POUR LES PREPARER, ET COMPOSITIONS PHARMACEUTIQUES QUI LES CONTIENNENT.

La présente invention a pour objet des dérivés d'(indényl--2)-2 imidazoline qui, sous forme de base libre, répondent à la formule générale I

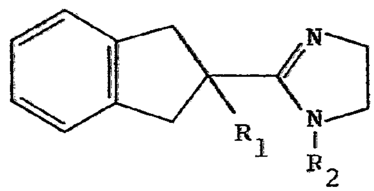

(I)

dans laquelle

$R_1$ est un atome d'hydrogène ou un groupe $(C_1-C_4)$alkyle, benzyle, phénéthyle ou méthoxy, et

$R_2$ est un atome d'hydrogène ou un groupe méthyle.

Ces composés peuvent exister également sous forme de sels d'addition à des acides, en particulier des acides pharmaceutiquemnt acceptables.

Le composé préféré de l'invention est celui dans la formule (I) duquel $R_1$ et $R_2$ sont chacun un atome d'hydrogène.

Les composés de l'invention peuvent être préparés selon des méthodes connues, à partir d'un dérivé de l'acide dihydro--2,3 1H-indène-2-carboxylique de formule II, dans laquelle R' est par exemple un groupe méthyle ou éthyle, par réaction avec une diamine de formule $R_2NHCH_2CH_2NH_2$ en présence de triméthylaluminium, selon le schéma suivant

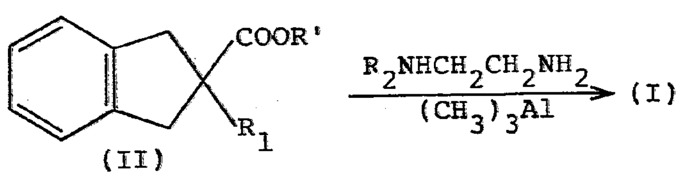


(II)

L'ester de départ (II) dans la formule duquel $R_1$ est l'hydrogène, peut être préparé par exemple selon la méthode de

Perkin et Revay, décrite dans J. Chem. Soc., 65, 228 (1894).

Pour obtenir des composés (II) dans la formule desquels $R_1$ est un groupe alkyle, benzyle ou phénéthyle, on effectue préalablement une alkylation par un composé de formule $R_1X$, X étant un groupe nucléofuge tel qu'un atome d'iode ou de brome, après avoir formé l'anion au moyen d'une base telle que le diisopropylamidure de lithium (LDA), selon le schéma suivant

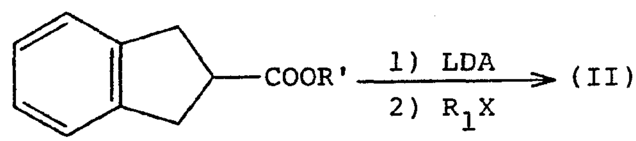


Enfin, pour obtenir les dérivés dans la formule (II) desquels $R_1$ est un groupe méthoxy, on peut utiliser l'acide hydroxy-2 dihydro-2,3 1H-indène-2-carboxylique, l'estérifier de manière connue avec un alcool R'OH, et le méthyler au moyen de iodométhane en présence d'hydrure de sodium, dans un solvant tel que le diméthylformamide, selon le schéma suivant

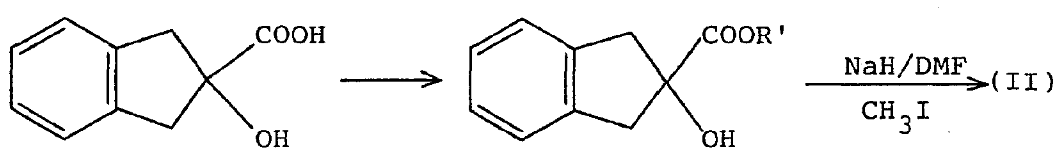


L'acide ci-dessus peut être préparé selon la méthode décrite dans C.R. Acad. Sci. Paris, 263, 325-9 (1966).

Les exemples suivants illustrent de façon plus détaillée la préparation des composés de l'invention. Les spectres IR et RMN confirment les structures des composés obtenus.

Exemple 1. (Dihydro-2,3 1*H*-indényl-2)-2 dihydro-4,5 1*H*-imidazole.

Dans un ballon tricol, on introduit sous argon 26 ml (0,0624 mole) de triméthylaluminium à 25 % dans de l'hexane, et 50 ml de toluène. A une température entre 0° et 5°C on ajoute goutte à goutte une solution des 3,8 g (0,0637 mole) d'éthylènediamine dans 20 ml de toluène. On laisse le mélange réactionnel revenir à la température ambiante puis on chauffe à 50°C. A cette température on ajoute goutte à goutte 7,05 g (0,04 mole) de dihydro-2,3 1*H*-indène-2-carboxylate de méthyle.
On distille l'hexane et chauffe le mélange à la température de reflux du toluène pendant 45 mn.
On hydrolyse en refroidissant avec 60 ml d'eau, ajoute 100 ml d'acétate d'éthyle, filtre le produit minéral, lave à l'eau la phase organique, sèche sur sulfate de magnésium et évapore.
On recueille un solide que l'on triture dans de l'éther de pétrole. Point de fusion : 122 - 123°C.

On prépare le fumarate dans de l'alcool isopropylique en mélangeant 6,4 g (0,0343 mole) de base et 3,7 g (0,0316 mole) d'acide fumarique. On évapore à sec et recristallise le fumarate dans de l'éthanol. Point de fusion : 202-203°C.

Exemple 2. (Propyl-2 dihydro-2,3 1*H*-indényl-2)-2 dihydro-4,5 1*H*-imidazole)

a) Dans une solution de 6,7 ml (0,048 mole) de diisopropylamine dans 50 ml de tétrahydrofuranne on introduit, à -78°C et sous argon, 30 ml (0,048 mole) d'une solution 1,6M de butyllithium dans l'hexane.
On agite 1 heure à -78°C puis on introduit 7,05 g (0,040 mole) de dihydro-2,3 1*H*-indène-2-carboxylate de méthyle en solution dans 30 ml de tétrahydrofuranne.
On continue l'agitation à -78°C pendant 1 heure, et on ajoute 34 g, soit 19,5 ml (0,2 mole) de iodopropane.
On agite 1 heure à -78°C, on laisse le mélange revenir à

température ambiante, on agite encore 1 heure, puis on jette le mélange sur de l'eau glacée, on l'extrait à l'éther, on le lave à l'eau, on le sèche sur sulfate de magnésium et on l'évapore. Il reste une huile que l'on distille à 160-170°C sous environ 2600 Pa (20 mmHg), puis purifie par passage sur une colonne de silice en éluant avec un mélange 60/40 de chlorure de méthylène/éther de pétrole.

b) Dans un ballon on introduit 19,7 ml, soit 3,38 g (0,047 mole) de triméthylaluminium (à 25% dans de l'hexane) en solution dans 50 ml de toluène puis, entre 0 et 5°C, sous argon, on introduit goutte à goutte 3,3 ml, soit 2,87 g (0,048 mole) d'éthylènediamine en solution dans 20 ml de toluène.

On chauffe le mélange à 50°C et on ajoute goutte à goutte 6,55 g (0,030 mole) de l'huile précédemment obtenue, dissoute dans 20 ml de toluène.

On chauffe le mélange pour distiller l'hexane, puis on le porte au reflux du toluène pendant 45 minutes.

On refroidit, et on ajoute, goutte à goutte, 45 ml d'eau. On ajoute de l'acétate d'éthyle, filtre le solide, sépare la phase organique, la lave à l'eau, la sèche sur sulfate de magnésium et l'évapore.

Le résidu, trituré dans l'éther de pétrole, filtré et séché, fond à 141-142°C.

On en prépare le fumarate dans une solution d'acide fumarique dans l'alcool isopropylique et on recristallise le sel précipité dans l'éthanol. Point de fusion · 186-187°C.

Exemple 3. (Dihydro-2,3 1<u>H</u>-indényl-2)-2 méthyl-1 dihydro-4,5 1<u>H</u>-imidazole.

Dans un ballon on introduit 16,4 ml, soit 2,81 g (0,039 mole) de triméthylaluminium (à 25% dans de l'hexane) en solution dans 50 ml de toluène puis, entre 0 et 5°C, sous argon, on introduit goutte à goutte 3,51 ml, soit 2,95 g (0,040 mole) de N-méthyl-éthylènediamine en solution dans 50 ml de toluène.

On chauffe le mélange à 50°C et on ajoute goutte à goutte 4,4 g (0,025 mole) de dihydro-2,3 1H-indène-2-carboxylate de méthyle.
On chauffe le mélange pour distiller l'hexane, puis on le porte au reflux du toluène pendant 45 minutes.
On le laisse refroidir et on ajoute, goutte à goutte, 37,5 ml d'eau.
On termine la préparation comme à l'exemple précédent, et l'on recueille le fumarate. Point de fusion : 171-172°C.

Exemple 4. (Méthoxy-2 dihydro-2,3 1H-indényl-2)-2 dihydro-4,5 1H-imidazole.

a) On estérifie 40,5 g (0,227 mole) d'acide hydroxy-2 dihydro-2,3 1H-indène-2-carboxylique en présence de 32,5 g (0,273 mole) de chlorure de thionyle en solution dans un excès d'éthanol, pendant 8 heures à la température du reflux. L'ester éthylique obtenu fond à 90-91°C.

b) Dans un erlenmeyer placé sous argon on introduit 2,1 g (0,044 mole) d'hydrure de sodium à 50 %, préalablement lavé au pentane, avec 50 ml de tétrahydrofuranne et 5 ml de diméthylformamide.
On ajoute goutte à goutte 8,25 g (0,04 mole) de l'ester hydroxylé précédemment préparé, ce qui provoque un dégagement d'hydrogène.
Au bout d'une heure d'agitation à température ambiante, on ajoute 12,5 ml, soit 28,4 g (0,2 mole) de iodométhane, et on agite encore 4 heures à température ambiante.
Puis on jette le mélange dans de l'eau acidifiée par de l'acide chlorhydrique, on l'extrait avec du chlorure de méthylène, on lave l'extrait à l'eau, on le sèche et on l'évapore. Il reste une huile que l'on purifie par un passage sur silice en éluant avec du chlorure de méthylène.

c) Dans un réacteur on introduit 40 ml de toluène et 20,7 ml (0,0495 mole) de triméthylaluminium en solution 1,6M dans l'hexane.
On ajoute goutte à goutte 3,04 g (0,0506 mole) d'éthylène-

diamine dissoute dans 20 ml de toluène, en maintenant la température entre 0 et 5°C.

On laisse revenir à température ambiante, puis on chauffe à 50°C, et on ajoute goutte à goutte 7 g (0,0317 mole) de l'huile précédemment obtenue en solution dans 30 ml de tétrahydrofuranne.

On chauffe pour distiller l'hexane, puis on porte au reflux du toluène pendant 45 minutes.

On laisse refroidir, on hydrolyse le mélange avec 45 ml d'eau et on ajoute de l'acétate d'éthyle.

On isole la phase organique, on la lave, on la sèche et on l'évapore.

On reprend le résidu et on en prépare le fumarate dans de l'alcool isopropylique. Point de fusion : 129-130°C.

Le tableau suivant illustre les structures et propriétés physiques de divers composés selon l'invention.

Tableau

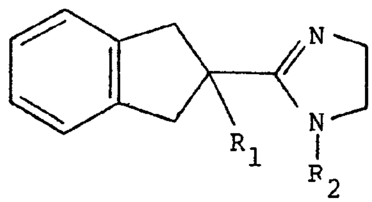

| Composé | $R_1$ | $R_2$ | F (°C, fumarate) |
|---|---|---|---|
| 1 | H | H | 202-203 |
| 2 | $-CH_2CH_3$ | H | 182-183 |
| 3 | $-CH_2CH_2CH_3$ | H | 186-187 |
| 4 | $-CH_2CH_2CH_2CH_3$ | H | 178-179 |
| 5 | $-CH_2C_6H_5$ | H | 158-159 |
| 6 | $-CH_2CH_2C_6H_5$ | H | 180-181 |
| 7 | H | $CH_3$ | 171-172 |
| 8 | $-OCH_3$ | H | 129-130 |

Les composés de l'invention ont été soumis à des essais pharmacologiques qui ont montré leur intérêt en tant que $\alpha_2$-antagonistes.

A cet effet les composés ont été étudiés dans le test de potentialité et de sélectivité des antagonistes à l'égard des récepteurs $\alpha_2$ in vitro.

La détermination de la valeur $pA_2$ à l'égard des effets inhibiteurs de la clonidine, $\alpha_2$-agoniste bien connu, a eu lieu sur le vas deferens du rat stimulé à une fréquence de 0,1 Hz en présence de 30 nM de prazosine et de 1 µM de cocaïne, selon la méthode décrite par G.M. Drew (European Journal of Pharmacology, 42, (1977) 123-130).

Les valeurs $pA_2$ des composés de l'invention vont de 6,0 à 8,3.

Leurs toxicités aiguës ont également été étudiées chez la souris par voie intrapéritonéale. Les doses létales $LD_{50}$ vont de 10 à 60 mg/kg.

Les composés de l'invention peuvent être utilisés pour le traitement de la dépression (soit seuls, soit en association avec un produit qui inhibe les mécanismes de captation neuronale), le traitement de l'hypotension et le traitement de l'ileum paralytique post-opératoire.

Les compositions pharmaceutiques peuvent selon l'invention être sous une forme appropriée pour l'administration par voie orale, rectale ou parentérale ; par exemple sous la forme de capsules, comprimés, granulés, gélules ou solutés liquides, sirops ou suspensions buvables, et contenir les excipients appropriés.

La posologie quotidienne peut aller de 1 à 30 mg/kg p.o.

Revendications pour les états contractants : BE, CH, DE, FR, GB, IT, LI, LU, NL et SE.

1. Composés répondant à la formule générale I

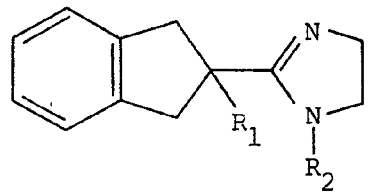

(I)

dans laquelle

$R_1$ est un atome d'hydrogène ou un groupe $(C_1-C_4)$alkyle, benzyle, phénéthyle ou méthoxy, et

$R_2$ est un atome d'hydrogène ou un groupe méthyle,

ainsi que leurs sels d'addition à des acides pharmaceutiquement acceptables.

2. Composé selon la revendication 1, caractérisé en ce que $R_1$ et $R_2$ sont chacun un atome d'hydrogène.

3. Procédé de préparation des composés selon la revendication 1, caractérisé en ce qu'on fait réagir un composé de formule II avec une diamine de formule $R_2NHCH_2CH_2NH_2$ en présence de triméthylaluminium, selon le schéma suivant

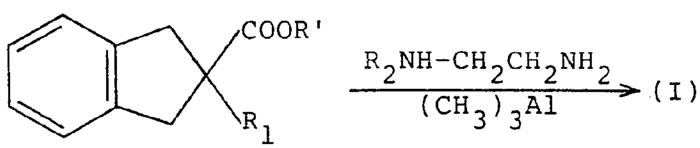


(II)

R' étant un groupe méthyle ou éthyle, et $R_1$ et $R_2$ étant tels que définis dans la revendication 1.

4. Médicament caractérisé en ce qu'il est constitué d'un composé selon la revendication 1.

5. Composition pharmaceutique caractérisée en ce qu'elle contient un composé selon la revendication 1, en association avec un excipient approprié.

Revendication pour l'état contractant : AT.

Procédé de préparation de composés répondant à la formule générale I

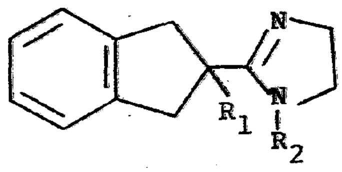

(I)

dans laquelle

$R_1$ est un atome d'hydrogène ou un groupe $(C_1-C_4)$alkyle, benzyle, phénéthyle ou méthoxy, et

$R_2$ est un atome d'hydrogène ou un groupe méthyle,

ainsi que de leurs sels d'addition à des acides pharmaceutiquement acceptables,

procédé caractérisé en ce qu'on fait réagir un composé de formule II avec une diamine de formule $R_2NHCH_2CH_2NH_2$ en présence de triméthylaluminium, selon le schéma suivant

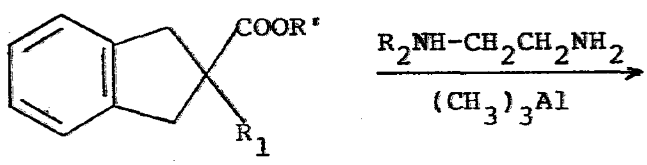


(I)

(II)

R' étant un groupe méthyle ou éthyle, et $R_1$ et $R_2$ étant tels que définis ci-dessus.